# EUROPEAN PATENT APPLICATION

(11) **EP 3 978 032 A1**
(43) Date of publication of application: **06.04.2022**
(21) Application number: 20813023.7
(22) Date of filing: 27.05.2020
(51) Int. Cl.: A61K 49/08, A61B 5/055

(54) **MACROPHAGE IMAGING AGENT**

(30) Priority: 31.05.2019 JP 2019103165
(71) Applicant: Kyoto University, Kyoto-shi, Kyoto 606-8501 (JP); National Cerebral and Cardiovascular Center, Suita-shi, Osaka 564-8565 (JP); JSR Corporation, Tokyo 105-8640 (JP)
(72) Inventor: KONDO, Teruyuki, Kyoto-shi, Kyoto 606-8501 (JP); KIMURA, Yu, Kyoto-shi, Kyoto 606-8501 (JP); SON, Aoi, Kyoto-shi, Kyoto 606-8501 (JP); MATSUDA, Tetsuya, Kyoto-shi, Kyoto 606-8501 (JP); IMAI, Hirohiko, Kyoto-shi, Kyoto 606-8501 (JP); KOSEKI, Hirokazu, Kyoto-shi, Kyoto 606-8501 (JP); AOKI, Tomohiro, Kishibe-shimmachi, Suita-shi, Osaka 564-8565 (JP); TAKASE, Katsuyuki, Tokyo 105-8640 (JP); MIYAJI, Masaaki, Tokyo 105-8640 (JP); TAMORI, Koji, Tokyo 105-8640 (JP); MASUDA, Takeaki, Tokyo 105-8640 (JP); KAI, Hirokazu, Tokyo 105-8640 (JP)
(74) Representative: Schön, Christoph
(86) International application number: PCT/JP2020/020850
(87) International publication number: WO 2020/241665

(57) **Abstract**

Composite particles, each of which is obtained by forming, on the surface of a gadolinium oxide-containing particle, a cover film that contains a polymer obtained by polymerizing a monomer component containing a monomer having an ethylenically unsaturated bond; a macrophage imaging agent which contains the composite particles; and a method for producing composite particles, wherein a monomer component containing a monomer having an ethylenically unsaturated bond and gadolinium oxide-containing particles are mixed with each other, and after emulsifying the thus-obtained monomer component-containing mixture in water in the presence of a surfactant and a polymerization initiator in water, the monomer component is polymerized, thereby forming cover films on the surfaces of the gadolinium oxide-containing particles.

## Description

### TECHNICAL FIELD

The present invention relates to a macrophage imaging agent. More particularly, the present invention relates to a macrophage imaging agent, and a composite particle used in the macrophage imaging agent and a method for producing the same.

### BACKGROUND ART

A magnetic resonance imaging method can be performed non-invasively without using radiation. Accordingly, the magnetic resonance imaging method has been used for molecular imaging of living bodies, and the like. As the molecular imaging of living bodies using the magnetic resonance imaging method, for example, macrophage imaging using polysaccharide-coated iron oxide particles as a macrophage imaging agent has been proposed (see, for example, Patent Literature 1).

However, when the polysaccharide-coated iron oxide particles are used as a macrophage imaging agent, magnetic resonance signals derived from the polysaccharide-coated iron oxide particles may be detected overlapping background noise derived from iron atoms in hemoglobin living bodies. Thus, the polysaccharide-coated iron oxide particles have a disadvantage that quantitative properties are impaired because of a need for image processing to erase background noise. In addition, the polysaccharide-coated iron oxide particles have a disadvantage that a load on living bodies is large.

In recent years, a macrophage imaging agent capable of easily imaging information relating to macrophages in living bodies and having a small load on living bodies has been desired.

### PRIOR ART LITERATURES

### PATENT LITERATURES

Patent Literature 1: Japanese Patent Unexamined Publication No. 2010-532223

### SUMMARY OF THE INVENTION

### PROBLEMS TO BE SOLVED BY THE INVENTION

The present invention has been made in view of the above-mentioned prior art. An object of the present invention is to provide a macrophage imaging agent capable of easily imaging information relating to macrophages in living bodies and having a small load on living bodies, and a composite particle used for the macrophage imaging agent and a method for producing the same.

### MEANS FOR SOLVING THE PROBLEMS

The present invention relates to:
(1) a macrophage imaging agent used for macrophage imaging by a magnetic resonance imaging method, including composite particles in which a coating film containing a polymer made by polymerizing a monomer component containing a monomer having an ethylenically unsaturated bond is formed on the surface of a gadolinium oxide-containing particle,
(2) a composite particle used in a macrophage imaging agent, including a gadolinium oxide-containing particle and a coating film containing a polymer made by polymerizing a monomer component containing a monomer having an ethylenically unsaturated bond, wherein the coating film is formed on the surface of the gadolinium oxide-containing particle, and
(3) a method for producing the composite particle according to the above item (2), including mixing a monomer component containing a monomer having an ethylenically unsaturated bond with a gadolinium oxide-containing particle, emulsifying the resulting monomer component-containing mixture in water in the presence of a surfactant and a polymerization initiator, and thereafter polymerizing the monomer component to form a coating film on the surface of the gadolinium oxide-containing particle.

### EFFECT OF THE INVENTION

According to the present invention, there is provided a macrophage imaging agent capable of easily imaging information relating to macrophages in living bodies and having a small load on living bodies, a composite particle used for the macrophage imaging agent, and a method for producing the composite particle.

### BRIEF DESCRIPTION OF THE DRAWINGS

Fig. 1 is a photograph substituted for a drawing, showing a result of observation of gadolinium oxide-containing particles obtained in Preparation Example 1.
Fig. 2 is a graph showing a particle size distribution of composite particles obtained in Example 1.
Fig. 3 shows photographs substituted for drawings, showing results of observation of the composite particles obtained in Example 1, wherein (A) is a photograph showing the result of observation of the composite particles obtained in Example 1, and (B) is a photograph showing the result of observation of an enlarged area surrounded by a broken line shown in the photograph of (A).
Fig. 4 is a graph showing a particle size distribution of composite particles obtained in Example 2.
Fig. 5 shows photographs substituted for drawings, showing T1 enhanced images in Test Example 1, wherein (A) is a photograph showing the T1 enhanced image in the presence of a test sample, and (B) is a photograph showing the T1 enhanced image in the presence of serum-containing test sample.
Fig. 6 shows photographs substituted for drawings, showing results of immunohistochemical staining in Preparation Example 4, wherein (A) is a photograph showing a result of immunohistochemical staining of normal carotid artery using anti-CD68 antibodies, (B) is a photograph showing a result of immunohistochemical staining of normal carotid artery using anti-α-SMA, (C) is a photograph showing a result of immunohistochemical staining of aneurysm lesions using anti-CD68 antibodies, and (D) is a photograph showing a result of immunohistochemical staining of aneurysm lesions using anti-α-SMA.

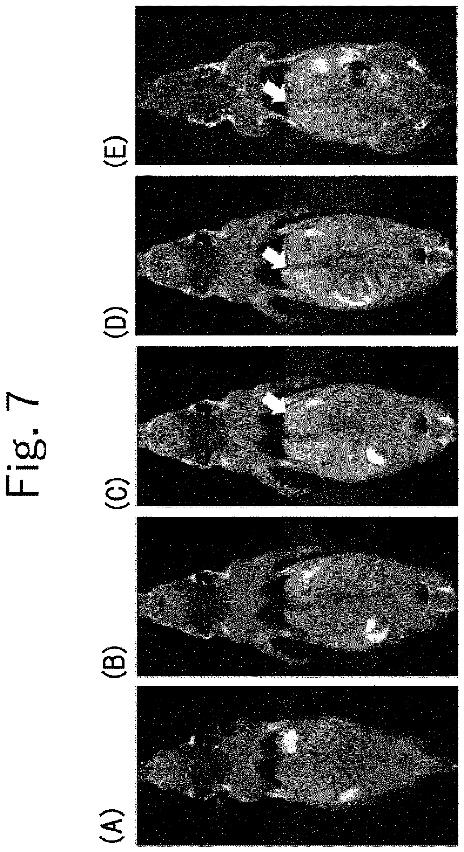

Fig. 7 shows photographs substituted for drawings showing T1-enhanced images of a mouse, wherein (A) is a photograph showing T1-enhanced images of a mouse prior to administration of a macrophage imaging agent obtained in Example 3, (B) is a photograph showing T1-enhanced images of a mouse after 30 minutes passed from the end of administration of the macrophage imaging agent obtained in Example 3, (C) is a photograph showing T1-enhanced images of a mouse after one hour passed from the end of administration of the macrophage imaging agent obtained in Example 3, (D) is a photograph showing T1-enhanced images of a mouse after 2 hours passed from the end of administration of the macrophage imaging agent obtained in Example 3, and (E) is a photograph showing T1-enhanced images of a mouse after 24 hours passed from the end of administration of the macrophage imaging agent obtained in Example 3.

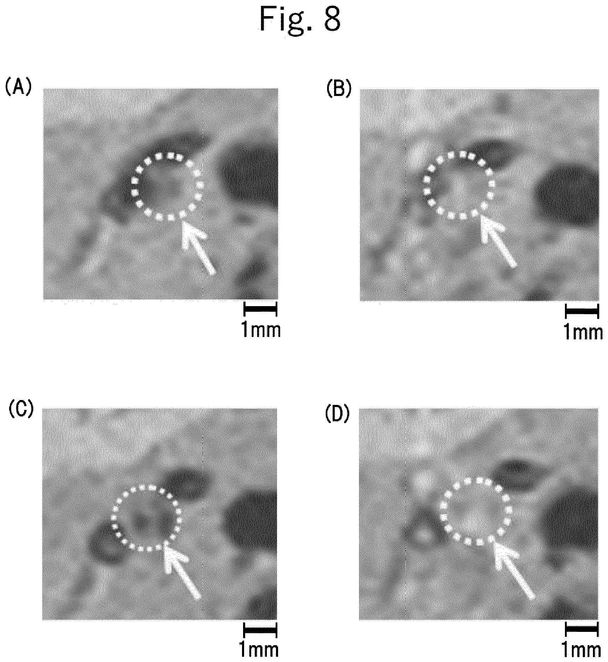

Fig. 8 shows photographs substituted for drawings showing T1-enhanced images of a rat, wherein (A) is photograph showing T1-enhanced images on a neck side of a carotid artery of a rat prior to administration of a macrophage imaging agent obtained in Example 4, (B) is a photograph showing T1-enhanced images on a neck side of a carotid artery of a rat after 24 hours passed from the end of administration of the macrophage imaging agent obtained in Example 4, (C) is a photograph showing T1-enhanced images on a dome side of a carotid artery of a rat prior to administration of a macrophage imaging agent obtained in Example 4, and (D) is a photograph showing T1-enhanced images on a dome side of a carotid artery of a rat after 24 hours passed from the end of administration of the macrophage imaging agent obtained in Example 4.
Fig. 9 is a graph showing a relation between a concentration of composite particles or a clinical contrast agent and a cell viability in an incubated sample obtained in Test Example 5.

### MODE FOR CARRYING OUT THE INVENTION

The macrophage imaging agent according to the present invention is a macrophage imaging agent used for macrophage imaging by a magnetic resonance imaging method, as stated above. The macrophage imaging agent includes composite particles in which a coating film containing a polymer made by polymerizing a monomer component containing a monomer having an ethylenically unsaturated bond is formed on the surface of a gadolinium oxide-containing particle.

According to the macrophage imaging agent of the present invention, the coating film containing a polymer made by polymerizing a monomer component containing a monomer having an ethylenically unsaturated bond is formed on the surface of a gadolinium oxide-containing. Accordingly, the macrophage imaging agent makes it possible to easily image an inside of living bodies and the like without carrying out, for example, a complicated operation such as image processing for erasing background noise. Moreover, the macrophage imaging agent exhibits an excellent effect that a load on the living body is small.

The composite particles can be produced, for example, by mixing a monomer component containing a monomer having an ethylenically unsaturated bond with gadolinium oxide-containing particles, emulsifying the resulting monomer component-containing mixture in water in the presence of a surfactant and a polymerization initiator, and thereafter polymerizing the monomer component to form a coating film on the surface of the gadolinium oxide-containing particle.

First, a monomer component containing a monomer having an ethylenically unsaturated bond is mixed with gadolinium oxide-containing particles, to prepare a monomer component-containing mixture.

The monomer having an ethylenically unsaturated bond includes, for example, a (meth)acrylate monomer, a carboxyl group-containing monomer, a styrene-based monomer, a halogen atom-containing unsaturated hydrocarbon monomer, a fatty acid vinyl ester monomer, an unsaturated nitrile monomer, and the like. The present invention is not limited only to those exemplified ones. These monomers having an ethylenically unsaturated bond can be used alone respectively or in combination of two or more thereof. Incidentally, the monomer having an ethylenically unsaturated bond used in the present invention is intended to mean a monomer having one ethylenically unsaturated bond in its molecule.

The (meth)acrylate monomer includes, for example, alkyl (meth)acrylates having an alkyl group of 1 to 18 carbon atoms, such as methyl (meth)acrylate, ethyl (meth)acrylate, butyl (meth)acrylate, 2-ethylhexyl (meth)acrylate and stearyl (meth)acrylate; cycloalkyl (meth)acrylates having a cycloalkyl group of 3 to 6 carbon atoms such as cyclohexyl (meth)acrylate; oxo group-containing (meth)acrylates such as ethylene glycol (meth)acrylate; fluoroalkyl (meth)acrylates having a fluoroalkyl group of 1 to 8 carbon atoms, such as trifluoroethyl (meth)acrylate and pentafluoropropyl (meth)acrylate; cyclic ether group-containing (meth)acrylates such as such as tetrahydrofurfuryl (meth)acrylate; (meth)acrylates having a reactive functional group, such as 2-bromoethyl (meth)acrylate, 3-(chloromethylsilyl)propyl (meth)acrylate, 2-(acetoacetyloxy)ethyl (meth)acrylate, allyl (meth)acrylate, 2-isocyanatoethyl (meth)acrylate, 2-azidoethyl (meth)acrylate and N-succinimide (meth)acrylate; and the like. The present invention is not limited only to those exemplified ones. These (meth)acrylate monomers can be used alone respectively or in combination of two or more thereof.

Incidentally, the "(meth)acrylate" used herein is intended to mean "acrylate" or "methacrylate", and the "(meth)acrylic" used herein is intended to mean "acrylic" or "methacrylic".

The carboxyl group-containing monomer includes, for example, aliphatic monomers having a carboxyl group and an ethylenically unsaturated double bond, such as (meth)acrylic acid, maleic acid, fumaric acid and itaconic acid. The present invention is not limited only to those exemplified ones. These carboxyl group-containing monomers can be used alone respectively or in combination of two or more thereof. Among the carboxyl group-containing monomers, (meth)acrylic acid is preferred.

The styrene-based monomer includes, for example, styrene, α-methylstyrene, p-methylstyrene, p-chlorostyrene, chloromethylstyrene, 4-(trimethoxysilyl)styrene, pentafluorostyrene and the like. The present invention is not limited only to those exemplified ones. These styrene-based monomers can be used alone respectively or in combination of two or more thereof.

The halogen atom-containing unsaturated hydrocarbon monomer includes, for example, vinyl chloride, vinylidene chloride, vinyl fluoride and the like. The present invention is not limited only to those exemplified ones. These halogen atom-containing unsaturated hydrocarbon monomers can be used alone respectively or in combination of two or more thereof. Among the halogen atom-containing unsaturated hydrocarbon monomers, vinyl chloride is preferred.

The fatty acid vinyl ester-based monomer includes, for example, vinyl acetate, vinyl propionate, vinyl chloroacetate, and the like. The present invention is not limited only to those exemplified ones. These fatty acid vinyl ester-based monomers can be used alone respectively or in combination of two or more thereof.

The unsaturated nitrile monomer includes, for example, (meth) acrylonitrile and the like. The present invention is not limited only to those exemplified ones.

Among the monomers having an ethylenically unsaturated bond, the (meth)acrylate monomer, the carboxyl group-containing monomer and the styrene-based monomer are preferred, and the alkyl (meth)acrylate having an alkyl group of 1 to 18 carbon atoms and styrene are more preferred, from the viewpoint of suppression of a load on the living body. The alkyl (meth)acrylate having an alkyl group of 1 to 4 carbon atoms is moreover preferred, the alkyl (meth)acrylate having an alkyl group of 1 to 3 carbon atoms is furthermore preferred, and methyl (meth)acrylate is still furthermore preferred, from the viewpoint of improvement in dispersion stability of the composite particles.

The content of the monomer having an ethylenically unsaturated bond in the monomer component is preferably 50% by mass or more, more preferably 70% by mass or more, further preferably 80% by mass or more, from the viewpoint of efficient production of the composite particles. The content of the monomer having an ethylenically unsaturated bond in the monomer component is 100% by mass or less, preferably 99% by mass or less, more preferably 97 % by mass or less, further preferably 95 % by mass or less, from the viewpoint of improvement in shape stability of the composite particles over time.

The monomer component may further contain a crosslinking monomer other than the monomer having an ethylenically unsaturated bond. The crosslinking monomer includes, for example, a divinyl monomer, a (meth)acrylate monomer having plural (meth)acryloyl groups, an allyl monomer having plural allyl groups, and the like. The present invention is not limited only to those exemplified ones. These crosslinking monomers can be used alone respectively or in combination of two or more thereof.

The divinyl monomer includes, for example, divinylbenzene, and the like. The present invention is not limited only to the exemplified one.

The (meth)acrylate monomer having plural (meth)acryloyl groups include, for example, di(meth)acrylate monomers such as ethylene glycol di(meth)acrylate, 1,6-hexandiol di(meth)acrylate and neopentyl glycol di(meth)acrylate; tri(meth)acrylate monomers such as trimethylolpropane tri(meth)acrylate and tetramethylolmethane tri(meth)acrylate; tetra(meth)acrylate monomers such as tetramethylolpropane tetra(meth)acrylate; and the like. The present invention is not limited only to those exemplified ones. These (meth)acrylate monomer having plural (meth)acryloyl groups can be used alone respectively or in combination of two or more thereof.

The allyl monomer having plural allyl groups includes, for example, diallyl phthalate, triallyl isocyanurate, and the like. The present invention is not limited only to those exemplified ones. These allyl monomers can be used alone respectively or in combination of two or more thereof.

Among the crosslinking monomers, the divinyl monomer and the (meth)acrylate monomer having plural (meth) acryloyl groups are preferred, divinylbenzene and trimethylolpropane tri(meth)acrylate are more preferred, and trimethylolpropane tri(meth)acrylate is further preferred, from the viewpoint of improvement in shape stability of the composite particles over time.

The content of the crosslinking monomer in the monomer component is preferably 1% by mass or more, more preferably 3% by mass or more, further preferably 5% by mass or more, from the viewpoint of improvement in shape stability of the composite particles over time. The content of the crosslinking monomer in the monomer component is preferably 50% by mass or less, more preferably 30% by mass or less, and further preferably 20% by mass or less, from the viewpoint of efficient production of the composite particles.

The monomer component may contain a monomer other than the monomer having an ethylenically unsaturated bond and the crosslinking monomer within a scope which would not inhibit an object of the present invention.

The gadolinium oxide-containing particles can be prepared, for example, by mixing a raw material compound with an unsaturated hydrocarbon compound having 5 to 24 carbon atoms and a fatty acid having 6 to 24 carbon atoms or its derivative, heating the resulting mixture to give a solution containing a gadolinium precursor of the fatty acid or its derivative, heating the resulting solution, mixing the solution with a polar solvent, and separating a precipitate from the resulting mixture. The gadolinium oxide-containing particles can be obtained in the form of the precipitate.

The raw material compound includes, for example, gadolinium nitrate, a hydrate of gadolinium nitrate, and the like. The present invention is not limited only to those exemplified ones. The hydrate of gadolinium nitrate includes, for example, gadolinium nitrate hexahydrate and the like, and the present invention is not limited only to the exemplified one.

The unsaturated hydrocarbon compound having 5 to 24 carbon atoms includes, for example, olefin hydrocarbon compounds having 5 to 24 carbon atoms, such as pentene, hexene, heptene, octene, nonene, decene, dodecene, tetradecene, pentadecene, hexadecene, heptadecene, octadecene, eicosene, docosene, tetracosane, and the like. The present invention is not limited only to those exemplified ones. These unsaturated hydrocarbon compounds having 5 to 24 carbon atoms can be used alone respectively or in combination of two or more thereof. Among the unsaturated hydrocarbon compounds having 5 to 24 carbon atoms, octadecene is preferred from the viewpoint of efficient production of the gadolinium oxide-containing particles.

The amount of the unsaturated hydrocarbon compound having 5 to 24 carbon atoms per mol of the raw material compound cannot be absolutely determined because the amount differs depending on the kind of the raw material compound, the kind of the unsaturated hydrocarbon compound having 5 to 24 carbon atoms, and the like. Accordingly, it is preferred that the amount is appropriately determined in accordance with the kind of the raw material compound, the kind of the unsaturated hydrocarbon compound having 5 to 24 carbon atoms, and the like. The amount of the unsaturated hydrocarbon compound having 5 to 24 carbon atoms per mol of the raw material compound is usually preferably 0.5 to 1.5 moles.

The fatty acid having 6 to 24 carbon atoms and its derivative includes, for example, saturated fatty acids such as hexanoic acid, heptanoic acid, octanoic acid, nonanoic acid, decanoic acid, dodecanoic acid, tetradecanoic acid, pentadecanoic acid, hexadecanoic acid, heptadecanoic acid, octadecanoic acid, eicosanoic acid, docosanoic acid and tetradocosanoic acid; unsaturated fatty acids such as crotonic acid, myristoleic acid, palmitoleic acid, sapienic acid, oleic acid, elaidic acid, vaccenic acid, gadoleic acid, eicosenoic acid, erucic acid and nervonic acid; aliphatic amines such as hexylamine, heptylamine, octylamine, nonylamine, decylamine, undecylamine, dodecylamine, tridecylamine, tetradecylamine, pentadecylamine, cetylamine, octadecylamine and oleylamine; and the like. The present invention is not limited only to those exemplified ones. These fatty acids having 6 to 24 carbon atoms and its derivative can be used alone respectively or in combination of two or more thereof. Among the fatty acid having 6 to 24 carbon atoms and its derivative, the unsaturated aliphatic acid having 6 to 24 carbon atoms is preferred, and oleic acid is more preferred, from the viewpoint of efficient production of the gadolinium oxide-containing particles.

The amount of the fatty acid having 6 to 24 carbon atoms or its derivative per mole of the raw material compound cannot be absolutely determined because the amount differs depending on the kind of the raw material compound, the kind of the fatty acid having 6 to 24 carbon atoms or its derivative, and the like. Accordingly, it is preferred that the amount is appropriately determined in accordance with the kind of the raw material compound, the kind of the fatty acid having 6 to 24 carbon atoms or its derivative, and the like. The amount of the fatty acid having 6 to 24 carbon atoms or its derivative per mol of the raw material compound is usually preferably 0.1 to 1 mole.

A solution containing a gadolinium precursor of the fatty acid or its derivative can be prepared by heating a mixture of the raw material compound, the unsaturated hydrocarbon compound having 5 to 24 carbon atoms and the fatty acid having 6 to 24 carbon atoms or its derivative in an inert gas atmosphere.

The heating temperature of the mixture is preferably 100°C or more, more preferably 110°C or more, from the viewpoint of efficient production of the gadolinium precursor of the fatty acid or its derivative. The heating temperature of the mixture is preferably 200°C or less, more preferably 190°C or less, from the viewpoint of efficient production of the gadolinium precursor of the fatty acid or its derivative, as well as the above. The heating period of time of the mixture is not particularly limited, and is usually preferably 0.5 to 4 hours, and more preferably 1 to 3 hours.

The mixture is heated in an inert gas atmosphere. The inert gas includes, for example, argon gas, nitrogen gas and the like, and the present invention is not limited only to those exemplified ones.

The solution containing the gadolinium precursor of the fatty acid or its derivative obtained in the above is then heated. The heating temperature of the solution is preferably 250°C or more, and more preferably 270°C or more, from the viewpoint of efficient production of the gadolinium oxide-containing particles. The heating temperature of the solution is preferably 350°C or less, and more preferably 330°C or less, from the viewpoint of efficient production of the gadolinium oxide-containing particles. The heating period of time of the solution is not particularly limited, and is usually preferably 0.5 to 20 hours, and more preferably 10 to 15 hours.

Next, the heated solution containing the gadolinium precursor of the fatty acid or its derivative is mixed with a polar solvent to give a mixture. The gadolinium oxide-containing particles that is composite particles of the gadolinium oxide-fatty acid or its derivative can be obtained by separating a precipitate from the resulting mixture.

The polar solvent includes, for example, water; aliphatic monohydric alcohols having 1 to 4 carbon atoms, such as methanol, ethanol, propanol and butanol; aliphatic ketone compounds such as acetone and methyl ethyl ketone; acetonitrile, dimethyl sulfoxide; and the like. The present invention is not limited only to those exemplified ones. These polar solvents can be used alone respectively, or in combination of two or more thereof within a scope which would not inhibit an object of the present invention. Among the polar solvents, the aliphatic monohydric alcohol and the aliphatic ketone compound are preferred, and methanol and acetone are more preferred, from the viewpoint of efficient production of the gadolinium oxide-containing particles.

The precipitate can be separated from the mixture, for example, by means of centrifugation or the like. The gadolinium oxide-containing particles can be obtained in the form of the precipitate.

A volume average particle diameter of the gadolinium oxide-containing particles cannot be absolutely determined because the volume average particle diameter differs depending on uses of a macrophage imaging agent and the like. Accordingly, it is preferred that the volume average particle diameter of the gadolinium oxide-containing particles is appropriately determined in accordance with the uses of a macrophage imaging agent and the like. The volume average particle diameter of the gadolinium oxide-containing particles is preferably 5 nm or more, and more preferably 8 nm or more, from the viewpoint of improvement in detection sensitivity of a magnetic resonance signal. The volume average particle diameter of the gadolinium oxide-containing particles is preferably 25 nm or less, more preferably 20 nm or less, and further preferably 15 nm or less, from the viewpoint of improvement in integration of the macrophage imaging agent to a macrophage.

The gadolinium oxide-containing particles can be used as a dispersion of the gadolinium oxide-containing particles. The dispersion of the gadolinium oxide-containing particles can be prepared, for example, by washing the gadolinium oxide-containing particles as occasion demands, and thereafter dispersing the gadolinium oxide-containing particles in a dispersion medium.

The gadolinium oxide-containing particles can be washed, for example, by mixing the gadolinium oxide-containing particles with a polar solvent to give a mixture, centrifugating the mixture to collect the gadolinium oxide-containing particles from the mixture. The polar solvent can be the same as the polar solvent which is used in mixing with the solution containing the gadolinium precursor of the fatty acid or its derivative.

The dispersion medium includes, for example, an aliphatic organic solvent and the like, and the present invention is not limited only to the exemplified ones. The aliphatic organic solvent includes, for example, aliphatic hydrocarbon compounds having 6 to 18 carbon atoms, such as hexane, heptane, octane, isooctane, decane, undecane, dodecane, tridecane, tetradecane, pentadecane, hexadecane, heptadecane and octadecane; aliphatic amines having 6 to 18 carbon atoms, such as hexylamine, heptylamine, octylamine, nonylamine, decylamine, undecylamine, dodecylamine, tridecylamine, tetradecylamine, pentadecylamine, hexadecylamine, heptadecyl amine and octadecylamine; aliphatic acids having 6 to 18 carbon atoms, such as hexanoic acid, heptanoic acid, octanoic acid, nonanoic acid, decanoic acid, dodecanoic acid, tetradecanoic acid, hexadecanoic acid, heptadecanoic acid and octadecanoic acid; and the like. The present invention is not limited only to the exemplified ones. These aliphatic organic solvents can be used alone respectively or in combination of two or more thereof. Among the aliphatic organic solvents, the aliphatic hydrocarbon compound having 6 to 18 carbon atoms is preferable, the aliphatic hydrocarbon compound having 6 to 10 carbon atoms is more preferable, hexane and heptane are moreover preferable, and hexane and heptane are furthermore preferable, from viewpoint of improvement in dispersion stability of the gadolinium oxide-containing particles.

The amount of the gadolinium oxide-containing particles per one L (liter) of the aliphatic organic solvent cannot be absolutely determined because the amount differs depending on uses of a macrophage imaging agent, and the like. Accordingly, it is preferred that the amount of the gadolinium oxide-containing particles is appropriately determined in accordance with the uses of the macrophage imaging agent, and the like. The amount of the gadolinium oxide-containing particles per one L of the aliphatic organic solvent is usually preferably 10 to 500 g, more preferably 50 to 250 g.

The dispersion of the gadolinium oxide-containing particles may contain another component other than the gadolinium oxide-containing particles and the dispersion medium within a scope which would not inhibit an object of the present invention. Another component includes, for example, a stabilizer for stably dispersing the gadolinium oxide-containing particles in the dispersion medium, and the like. The present invention is not limited only to those exemplified ones.

The stabilizer includes, for example, a surfactant, an acid group-containing compound, an amino group-containing compound, a silane group-containing compound, a titanium atom-containing compound and the like, and the present invention is not limited only to those exemplified ones. These stabilizers can be used alone respectively or in combination of two or more thereof.

The surfactant includes, an anionic surfactant, a cationic surfactant, a nonionic surfactant, an amphoteric surfactant and a polymeric surfactant. These surfactants can be used alone respectively or in combination of two or more thereof.

The anionic surfactant includes, for example, an alkali metal salt or an ammonium salts of an alkyl sulfate having an alkyl group of 4 to 16 carbon atoms, such as sodium dodecyl sulfate, potassium dodecyl sulfate and ammonium dodecyl sulfate; an alkali metal salt or an ammonium salt of an alkylbenzene sulfuric acid having an alkyl group of 4 to 16 carbon atoms; an alkali metal salt of a dialkylsulfosuccinic acid; an alkali metal salt of an alkyl diphenyl ether disulfonic acid having an alkyl group of 4 to 16 carbon atoms; a sulfate salt or a phosphate salt of a polyoxyethylene alkyl ether having an alkyl group of 4 to 16 carbon atoms; a sulfate salt or a phosphate salt of a polyoxyethylene alkylphenyl ether having an alkyl group of 4 to 16 carbon atoms; a folmaldehyde condensate of a sodium naphthalene sulfonate; and the like. The present invention is not limited only to those exemplified ones.

In addition, as the anionic surfactant, commercially available ones can be used. Its examples include LATEMUL S-180A [manufactured by Kao Corporation, trade name], ELEMINOR JS-2 [manufactured by Sanyo Kasei Co., Ltd., trade name], AQUALON HS-10 [manufactured by Daiichi Kogyo Seiyaku Co., Ltd., trade name], ADEKA REASOAP SE-10N [manufactured by ADEKA Corporation, trade name], and the like. The present invention is not limited only to those exemplified ones.

The cationic surfactant includes, for example, monoalkyltrimethylammonium chlorides such as hexadecyltrimethylammonium chloride and cetyltrimethylammonium bromide; quaternary ammonium salts such as alkylpyridinium salts such as hexadecylpyridinium chloride; and the like. The present invention is not limited only to those exemplified ones.

The nonionic surfactant includes, for example, fatty acid monoglyceride, polyoxyethylene alkyl ether, polyoxyethylene alkyl aryl ether, polyethylene glycol fatty acid ester, and the like. The present invention is not limited only to those exemplified ones.

In addition, As the nonionic surfactant, commercially available ones can be used. Its examples include Triton X-100 (manufactured by Union Carbide, trade name), Triton X-114 (manufactured by Union Carbide, trade name), Triton X-305 (manufactured by Union Carbide, trade name), Triton N-101 (manufactured by Union Carbide, trade name), Tween 20 (manufactured by Imperial Chemical Industries [ICI], trade name), Tween 40 (manufactured by Imperial Chemical Industries [ICI], trade name), Tween 60 (manufactured by Imperial Chemical Industries [ICI], trade name), Tween 80 (manufactured by Imperial Chemical Industries [ICI], trade name), Tween 85 (manufactured by Imperial Chemical Industries [ICI], trade name), Brij 35 (manufactured by Imperial Chemical Industries [ICI], trade name), Brij 58 (manufactured by Imperial Chemical Industries [ICI], trade name), Brij 76 (manufactured by Imperial Chemical Industries [ICI], trade name), Brij 98 (manufactured by Imperial Chemical Industries [ICI], trade name), Nonidet P-40 (manufactured by SHELL, trade name), Igepol CO530 (manufactured by RHONE POULENC, trade name), Igepol CO630 (Manufactured by RHONE POULENC, trade name), Igepol CO720 (manufactured by RHONE POULENC, trade name), Igepol CO730 (manufactured by RHONE POULENC, trade name), and the like. The present invention is not limited only to those exemplified ones.

The polymeric surfactant includes, for example, polyvinyl alcohol, and the like, and the present invention is not limited only to the exemplified one.

Among the surfactants, the anionic surfactant is preferred, the alkali metal salt or ammonium salt of the alkyl sulfate ester having an alkyl group of 4 to 16 carbon atoms are more preferred, sodium dodecyl sulfate, potassium dodecyl sulfate and ammonium dodecyl sulfate are moreover preferred, and sodium dodecyl sulfate is further preferred, from viewpoint of improvement in dispersion stability of the gadolinium oxide-containing particles.

The acid group-containing compound includes, for example, a carboxylic acid compound having one carboxyl group, a carboxylic acid compound having two or more carboxyl groups, a sulfonic acid compound having one sulfo group, a sulfonic acid compound having two or more sulfo groups, a sulfonic acid compound having two or more sulfo groups, a sulfocarboxylic acid compound having one carboxyl group and one sulfo group, a Bronsted acid and the like, and the present invention is not limited only to those exemplified ones. These acid group-containing compounds can be used alone respectively or in combination of two or more thereof.

The carboxylic acid compound having one carboxyl group includes, for example, aliphatic saturated monocarboxylic acids such as formic acid, acetic acid, propionic acid, butyric acid, isobutyric acid, valeric acid, caproic acid, enanthic acid, caprylic acid, pelargonic acid,
lauric acid, myristic acid, stearic acid and behenic acid; aliphatic unsaturated monocarboxylic acids such as acrylic acid, methacrylic acid and oleic acid; oxyaliphatic monocarboxylic acids such as glycolic acid, lactic acid and tartaric acid; alicyclic monocarboxylic acids such as cyclopentanecarboxylic acid and cyclohexanecarboxylic acid; aromatic monocarboxylic acids such as benzoic acid, cinnamic acid and naphthoic acid; oxyaromatic monocarboxylic acids such as salicylic acid and mandelic acid; trifluoroacetic acid; and the like. The present invention is not limited only to those exemplified ones.

The carboxylic acid compound having two or more carboxyl groups includes, for example, aliphatic saturated polycarboxylic acids having 2 to 30 carbon atoms, such as oxalic acid, malonic acid, succinic acid, glutaric acid, adipic acid, pimelic acid, suberic acid, azelaic acid, sebacic acid, propane-1,2,3-tricarboxylic acid, citric acid and dodecanedioic acid; aliphatic unsaturated polycarboxylic acids having 2 to 30 carbon atoms, such as maleic acid, fumaric acid and itaconic acid; aromatic polycarboxylic acids having 8 to 30 carbon atoms, such as phthalic acid, isophthalic acid, terephthalic acid, trimellitic acid and pyromellitic acid; alicyclic polycarboxylic acids having 5 to 30 carbon atoms, such as cyclobutene-1,2-dicarboxylic acid, cyclopentene-1,2-dicarboxylic acid, furan-2,3-dicarboxylic acid, bicyclo[2.2.1]hept-2-ene-2,3-dicarboxylic acid and bicyclo[2.2.1]hept-2,5-diene-2,3-dicarboxylic acid; and the like. The present invention is not limited only to those exemplified ones.

The sulfonic acid compound having one sulfo group includes, for example, aliphatic monosulfonic acids such as methanesulfonic acid, ethanesulfonic acid, propane sulfonic acid, butanesulfonic acid, hexansulfonic acid, decansulfonic acid, undecanesulfonic acid and dodecanesulfonic acid; aromatic monosulfonic acids such as benzenesulfonic acid, p-toluenesulfonic acid, o-toluenesulfonic acid, m-toluenesulfonic acid, 4-dodecylbenzenesulfonic acid, 4-octylbenzenesulfonic acid and naphthalene sulfonic acid; trifluoromethanesulfonic acid; and the like. The present invention is not limited only to those exemplified ones.

The sulfonic acid compound having two or more sulfo groups includes, for example, aliphatic polysulfonic acids having 1 to 30 carbon atoms, such as methionic acid, 1,1-ethanedisulfonic acid, 1,2-ethanedisulfonic acid, 1,1-propanedisulfonic acid, 1,3-propanedisulfonic acid and polyvinylsulfonic acid; aromatic polysulfonic acids having 6 to 30 carbon atoms such as m-benzenedisulfonic acid, 1,4-naphthalenedisulfonic acid, 1,5-naphthalenedisulfonic acid, 1,6-naphthalenedisulfonic acid, 2,6-naphthalenedisulfonic acid, 2,7-naphthalenedisulfonic acid and sulfonated polystyrene; bis(fluorosulfonyl)imide, bis(trifluoromethanesulfonyl)imide; and the like. The present invention is not limited only to those exemplified ones.

The sulfocarboxylic acid compound having one carboxyl group and one sulfo group include, for example, sulfocarboxylic acid acids having 2 to 30 carbon atoms, such as sulfoacetic acid and sulfosuccinic acid; sulfoaromatic carboxylic acid acids having 7 to 30 carbon atoms, such as o-sulfobenzoic acid, m-sulfobenzoic acid, p-sulfobenzoic acid, 2,4-disulfobenzoic acid, 3-sulfophthalic acid, 3,5-disulfophthalic acid, 4-sulfoisophthalic acid, 2-sulfoterephthalic acid, 2-methyl-4-sulfo benzoic acid, 2-methyl -3,5-disulfobenzoic acid, 4-propyl-3-sulfo benzoic acid, 4-isopropyl-3-sulfobenzoic acid, 2,4,6-trimethyl-3-sulfobenzoic acid, 2-methyl-5-sulfoterephthalic acid, 5-methyl-4-sulfoisophthalic acid, 5-sulfosalicylic acid and 3-oxy-4-sulfo benzoic acid; and the like. The present invention is not limited only to those exemplified ones.

The Bronsted acid includes, for example, tetrafluoroboric acid, trifluoromethyl trifluoroborate, hydrofluoric acid, hydrochloric acid, hydrobromic acid, hydroiodic acid, meta-arsenious acid, arsenious acid, arsenic acid, hexafluoro arsenic acid, boric acid, hypochlorous acid, carbonic acid, hydrogen cyanide, cyanic acid, hypochlorous acid, chlorous acid, perchloric acid, chromic acid, hypoiodous acid, iodic acid, molybdic acid, hydrazoic acid, hyponitrous acid, nitrous acid, nitric acid, isothiocyanic acid, hydrogen peroxide, phosphinic acid, phosphonic acid, phosphoric acid, pyrophosphoric acid, tripolyphosphate, hexafluorophosphate, hydrogen sulfide, sulfuric acid, thiosulfuric acid, selenious acid, selenic acid, orthosilicic acid, fluoroantimonic acid, tellurous acid, telluric acid, vanadic acid, tungstic acid, and the like. The present invention is not limited only to those exemplified ones.

The amino group-containing compound includes, for example, fluorine-containing amines such as perfluorotriethylamine, perfluorotripropylamine, perfluorotributylamine, perfluorotripentyl amine, perfluorohexylamine, bis(2,2-trifluoroethyl) amine, 1H,1H-perfluorooctylamine, pentafluoroaniline, 3,5-bis(trifluoromethyl)aniline and the like, and the present invention is not limited only to those exemplified ones. These amino group-containing compounds can be used alone respectively or in combination of two or more thereof.

The silane group-containing compound includes, for example, isobutyltrimethoxysilane, vinyltrichlorosilane, vinyltrimethoxysilane, vinyltris((β-methoxyethoxy)silane, β-(3,4-epoxycyclohexyl)ethyltrimethoxysilane, γ-glycidoxypropyltrimethoxysilane, γ-(meth)acryloyloxypropyltrimethoxysilane, N-β(aminoethyl)-γ-aminopropylmethyldimethoxysilane, N-β(aminoethyl)-γ-aminopropyltrimethoxysilane, dodecyltrimethoxysilane, hexyltrimethoxysilane, methyltrimethoxysilane, methyltriethoxysilane, phenyltrimethoxysilane, dodecyltrichlorosilane, hexyltrichlorosilane, methyltrichlorosilane, phenyltrichlorosilane and the like, and the present invention is not limited only to those exemplified ones. These silane group-containing compounds can be used alone respectively or in combination of two or more thereof.

The titanium atom-containing compound includes, for example, titanium triisostearoylisopropoxide, (2-n-butoxycarbonylbenzoyloxy)tributoxytitanium, titanium acetylacetonate, iso-butoxy titanium ethyl acetoacetate, tetraisopropyl titanate, tetra-n-butyl titanate and the like, and the present invention is not limited only to those exemplified ones. These titanium atom-containing compounds can be used alone respectively or in combination of two or more thereof.

The content of the stabilizer in the dispersion of the gadolinium oxide-containing particles cannot be absolutely determined because the content of the stabilizer differs depending on kinds of the stabilizer and the like. Accordingly, it is preferred that the content of the stabilizer is appropriately determined in accordance with the kinds of the stabilizer and the like.

The dispersion of the gadolinium oxide-containing particles can be used as it is. Alternatively, a part of the dispersion medium contained in the dispersion of the gadolinium oxide-containing particles can be replaced with another dispersion medium.

The amount of the monomer component per 100 parts by mass of the gadolinium oxide-containing particles is preferably 10 parts by mass or more, more preferably 50 parts by mass or more, furthermore preferably 100 parts by mass or more, and even more preferably 150 parts by mass or more, from the viewpoint of efficient covering of the gadolinium oxide-containing particles with the polymer. The amount of the monomer component per 100 parts by mass of the gadolinium oxide-containing particles is preferably 1000 parts by mass or less, more preferably 900 parts by mass or less, furthermore preferably 800 parts by mass or less, even more preferably 700 parts by mass or less, from the viewpoint of improvement in detection sensitivity of a magnetic resonance signal.

Next, the resulting monomer component-containing mixture is emulsified in water in the presence of a surfactant and a polymerization initiator, to give an emulsion.

The surfactant can be the same as the surfactant which is used in the stabilizer of the dispersion of the gadolinium oxide-containing particles. The surfactant may have a reactive group polymerizable with the monomer component. The reactive group includes, for example, ethylenically unsaturated groups such as vinyl group, allyl group and (meth)acryloyl group, and the like. The present invention is not limited only to those exemplified ones.

The amount of the surfactant per 100 parts by mass of the monomer component-containing mixture cannot be absolutely determined, because the amount differs depending on the kind of the monomer, the amount of the gadolinium oxide-containing particles, a desired particle diameter of the composite particle, and the like. Accordingly, it is preferred that the amount of the surfactant is appropriately determined in accordance with the kind of the monomer, the amount of the gadolinium oxide-containing particles, a desired particle diameter of the composite particle, and the like. The amount of the surfactant per 100 parts by mass of the monomer component-containing mixture is usually preferably 0.01 parts by mass or more, and more preferably 0.1 parts by mass or more, from the viewpoint of efficient preparation of the emulsion. The amount of the surfactant per 100 parts by mass of the monomer component-containing mixture is usually preferably 20 parts by mass or less, and more preferably 10 parts by mass or less, from the viewpoint of efficient preparation of the emulsion, as well as mentioned above.

The polymerization initiator includes, for example, azo compounds such as 2,2'-azobisisobutyronitrile, 2,2'-azobis-(2-methylpropanenitrile), 2,2'-azobis-(2,4-dimethylpentanenitrile), 2,2'-azobis-(2-methylbutanenitrile), 1,1'-azobis-(cyclohexanecarbonitrile), 2,2'-azobis-(2,4-dimethyl-4-methoxyvaleronitrile), 2,2'-azobis-(2,4-dimethylvaleronitrile) and 2,2'-azobis-(2-amidinopropane)hydrochloride; peroxides such as benzoyl peroxide, cumene hydroperoxide, hydrogen peroxide, acetyl peroxide, lauroyl peroxide, tert-butyl peroxyoctoate, and α-cumyl peroxypivalate; persulfates such as ammonium persulfate; and the like. The present invention is not limited only to those exemplified ones. These polymerization initiators can be used alone respectively or in combination of two or more thereof.

The amount of the polymerization initiator per 100 parts by mass of the monomer component-containing mixture cannot be absolutely determined because the amount differs depending on the kind of the monomer, the amount of the monomer and the like. Accordingly, it is preferred that the amount is appropriately determined in accordance with the kind of the monomer, the amount of the monomer and the like. The amount of the polymerization initiator per 100 parts by mass of the monomer component-containing mixture is usually 0.1 to 20 parts by mass, and more preferably 1 to 10 parts by mass.

After the monomer component-containing mixture is mixed with the surfactant, the polymerization initiator and water, the monomer component-containing mixture can be emulsified, for example, by using a shear mixing apparatus such as an ultrasonic disperser. The shear mixing apparatus includes, for example, an ultrasonic emulsification apparatus such as a probe-type ultrasonic disperser, and the present invention is not limited only to the exemplified one.

It is preferred that the monomer component-containing mixture is emulsified in water so that the volume average particle diameter of the composite of the monomer component and the gadolinium oxide-containing particle can be the same as the objective volume average particle diameter of the composite particles.

When the ultrasonic disperser is used, the output of the ultrasonic waves is preferably 5W or more, and more preferably 10W or more, from the viewpoint of efficient covering of the gadolinium oxide-containing particles with the polymer, and is preferably 200W or less, and more preferably 150W or less, from the viewpoint of being able to easily image the inside of living bodied and efficient production of a macrophage imaging agent showing a low load on living bodies. The irradiation of ultrasonic waves can be performed once or multiple times. The period of time for irradiating ultrasonic waves per irradiation of the ultrasonic waves cannot be absolutely determined because the period of time for irradiating ultrasonic waves differs depending on output of the ultrasonic waves and the like. Accordingly, it is preferred that the period of time for irradiating ultrasonic waves is determined in accordance with the output of the ultrasonic waves and the like. It is preferred that the period of time for irradiating ultrasonic waves per irradiation of the ultrasonic waves is preferably 10 seconds or more, more preferably 30 seconds or more, and furthermore preferably one minute or more, from the viewpoint of sufficient emulsifying of the monomer component-containing mixture, and formation of composite particles having a small volume average particle diameter, and is preferably 10 minutes or less, more preferably 5 minutes or less, and furthermore preferably 3 minutes or less, from the viewpoint of efficient adhesion of the monomer component to the gadolinium oxide-containing particles.

Next, the monomer component is polymerized, to form a coating of the polymer being prepared by polymerizing the monomer component on the surface of the gadolinium oxide-containing particles.

The polymerization of the monomer component can be carried out in one stage or in multiple stages. Thus, the coating film of the composite particles may have a single layer structure, or may have a multilayer structure. The polymerization temperature of the monomer component cannot be absolutely determined, because the polymerization temperature differs depending on the kind of the monomers included in the monomer component, and the like. Accordingly, it is preferred that the polymerization temperature is appropriately determined in accordance with the kind of the monomers included in the monomer component, and the like. The polymerization temperature of the monomer component is usually preferably 40 to 100°C, and more preferably 50 to 95°C. The polymerization period of time for polymerizing the monomer component is not particularly limited, and is usually preferably 1 to 50 hours, and more preferably 2 to 24 hours.

The content of the polymer in the composite particles is preferably 5% by mass or more, more preferably 8% by mass or more, and furthermore preferably 10% by mass or more, from the viewpoint of suppression of a load on living bodies, and is preferably 99% by mass or less, more preferably 97% by mass or less, furthermore preferably 95% by mass or less, from the viewpoint of improvement in integration of the macrophage imaging agent to a macrophage and improvement in detection sensitivity of a magnetic resonance signal.

The content of the gadolinium oxide-containing particles in the composite particles cannot be absolutely determined because the content differs depending on uses of the macrophage imaging agents and the like. Accordingly, it is preferred that the content of the gadolinium oxide-containing particles is appropriately determined in accordance with the uses of the macrophage imaging agents and the like. The content of the gadolinium oxide-containing particles in the composite particles is preferably 1% by mass or more, more preferably 3% by mass or more, and furthermore preferably 5% by mass or more, from the viewpoint of improvement in integration of the macrophage imaging agent to a macrophage and improvement in detection sensitivity of a magnetic resonance signal, and is preferably 95 % by mass or less, more preferably 92 % by mass or less, furthermore preferably 90 % by mass or less, from the viewpoint of suppression of a load on living bodies.

The volume average particle diameter of the composite particles cannot be cannot be absolutely determined, because the volume average particle diameter differs depending on uses of the macrophage imaging agents and the like. Accordingly, it is preferred that the volume average particle diameter of the composite particles is appropriately determined in accordance with the uses of the macrophage imaging agents, and the like. The volume average particle diameter of the composite particles is preferably 10 nm or more, more preferably 50 nm or more, and furthermore preferably 80 nm or more, from the viewpoint of improvement in detection sensitivity of a magnetic resonance signal, and is preferably 200 nm or less, more preferably 190 nm or less, furthermore preferably 150 nm or less, still more preferably 100 nm or less, from the viewpoint of suppression of a load on living bodies and improvement in integration of the macrophage imaging agent to a macrophage.

The macrophage imaging agent of the present invention can be composed only of the composite particles. Alternatively, the macrophage imaging agent may contain another component other than the composite particles within a scope which would not hider an object of the present invention. Another component includes, for example, a dispersion medium for dispersing the composite particles, a stabilizer and the like, and the present invention is not limited only to those exemplified ones.

The dispersion medium includes, for example, water; alcohol-based organic solvents such as methanol, ethanol, n-propanol, isopropanol and ethylene glycol; ketone-based organic solvents such as methyl isobutyl ketone and cyclohexanone; ester-based organic solvents such as ethyl acetate, propyl acetate and propylene glycol monomethyl ether acetate; ethers such as ethylene glycol monomethyl ether and diethylene glycol monobutyl ether; hydrocarbon-based organic solvents such as toluene, xylene, ethylbenzene, trimethylbenzene, hexane, cyclohexane, methylcyclohexane, ethylcyclohexane and a mineral spirit; halogenated hydrocarbon compounds such as dichloromethane and chloroform; amide-based organic solvents such as dimethylformamide, N,N-dimethylacetamide and N-methylpyrrolidone; oils such as a mineral oil, a vegetable oil, a wax oil and a silicone oil; and the like. The present invention is not limited only to those exemplified ones. Among the solvents, the alcohol-based organic solvent, the ketone-based organic solvent and the hydrocarbon-based organic solvent are preferable, and the ketone-based organic solvent and the hydrocarbon-based organic solvent are more preferable, from the viewpoint of ease of handling. These solvents can be used alone respectively or in combination of two or more thereof.

The stabilizer can be the same as the stabilizer which are used in the dispersion of the gadolinium oxide-containing particles.

The content of the composite particles in the macrophage imaging agent cannot be absolutely determined, because the content differs depending on uses of a macrophage imaging agent, and the like. Accordingly, it is preferred that the content of the composite particles in the macrophage imaging agent is appropriately determined in accordance with the uses of a macrophage imaging agent, and the like. The content of the composite particles in the macrophage imaging agent is preferably 0.01% by mass or more, more preferably 0.1% by mass or more, from the viewpoint of improvement in detection sensitivity of the magnetic resonance signal. The upper limit of the content of the composite particles in the macrophage imaging agent is 100% by mass. When the macrophage imaging agent contains another component, it is preferred that the upper limit of the content of the composite particles in the macrophage imaging agent is appropriately determined in accordance with the kind of another component, and the like within a scope which would not inhibit an object of the present invention.

The macrophage imaging agent of the present invention is excellent in integration to a macrophage. Accordingly, the macrophage imaging agent can be used, for example, in image diagnosis such as a disease involving a macrophage. The disease involving a macrophage includes, for example, inflammation; tumor; cardiac diseases such as myocardial infarction; cerebrovascular diseases such as stroke and cerebral infarction; arteriosclerosis; diabetes mellitus; neurological disorders such as Alzheimer's disease; and the like. The present invention is not limited only to those exemplified ones.

As described above, the macrophage imaging agent according to the present invention can easily image information on a macrophage in living bodies, and does not impose a heavy load on living bodies. Accordingly, the macrophage imaging agent is expected to be suitably used for image diagnosis of a disease in which macrophages are involved, and the like.

### EXAMPLES

The present invention will be more specifically described with reference to the following working examples. The present invention is not limited only to the working examples. The meaning of each abbreviation is as follows:

### <Description of abbreviations>

EDTA: Ethylenediaminetetraacetic acid
FBS: Fetal bovine serum
PBS: Phosphate buffered saline
TR: Repeat time
TE: Echo time
FOV: Imaging field
NA: Average number

### Preparation Example 1

In a flat bottom reaction tube, 450 mg (10 mmol) of gadolinium nitrate hexahydrate and a glass-coated stirrer were placed, and thereafter, a cooler was attached to the top of the flat bottom reaction tube. A series of procedures including decompression in the flat bottom reaction tube, introducing of argon gas to the flat bottom reaction tube was repeated three times, to replace the atmosphere in the flat bottom reaction tube with argon gas. While argon gas was introduced into the flat bottom reaction tube, 563 mg (2 mmol) of oleic acid and 2500 mg (10 mmol) of 1-octadecene were added to the gadolinium nitrate hexahydrate in the flat bottom reaction tube, to give a mixture. The mixture obtained in the above was heated at 110°C for 2 hours with stirring in an argon gas atmosphere, to give a yellow solution. The yellow solution obtained in the above was heated at 320°C for 14 hours with stirring to give a brown solution. The brown solution obtained in the above was cooled to room temperature with stirring, and thereafter, the brown solution was added to a centrifuge tube having a volume of 50mL. To the brown solution, 20 mL of methanol and 20 mL of acetone were added, and the contents in the centrifuge tube were vigorously shaken. Thereafter, the resulting mixture was conducted to centrifugation at 1873 ×g for 30 minutes, to separate into a light brown supernatant and a black precipitate. A series of procedures including adding 20 mL of methanol and 20 mL of acetone to the black precipitate obtained in the above, shaking the resulting mixture, and centrifugating the mixture at 1873 ×g for 30 minutes was repeated 6 times, to wash the black precipitate. The washed black precipitate was dispersed in 10 mL of hexane, to give a dispersion of the gadolinium oxide-containing particles.

The dispersion of the gadolinium oxide-containing particles obtained in the above was diluted 1000 times with a dilution solvent having a composition of 1M nitric acid and 1% by mass of polyoxyethylene (10) octylphenyl ether, to give a measurement sample. The concentration of the gadolinium oxide-containing particles in the measurement sample was determined by using a Polarized Zeeman Atomic Absorption Spectrophotometer Z-2710 made by Hitachi High-Tech Corporation, and the concentration of the gadolinium oxide-containing particles in the dispersion of the gadolinium oxide-containing particles was determined. As a result, the concentration of the gadolinium oxide-containing particles in the dispersion was about 100 mM.

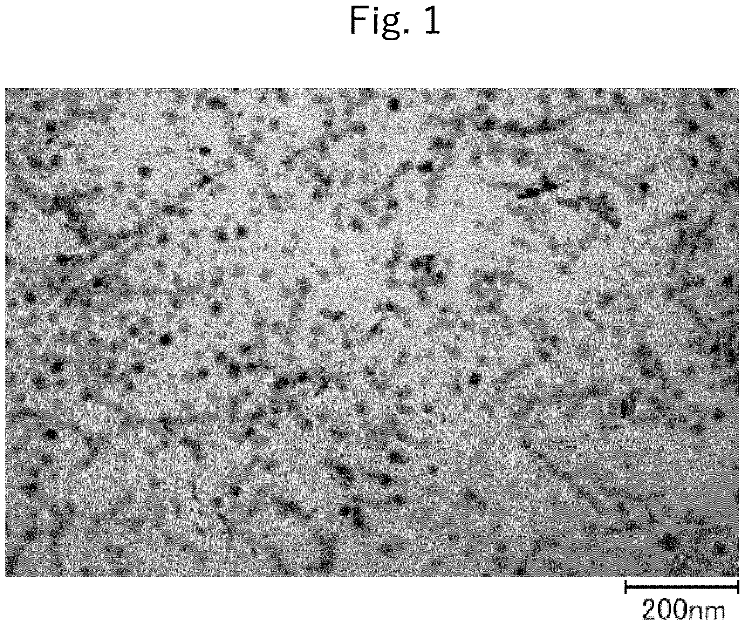

The gadolinium oxide-containing particles contained in the measurement sample were observed at an acceleration voltage of 100 kV by using a transmission electron microscope [made by JEOL Ltd., trade name: JEM -1400]. The results of observation of the gadolinium oxide-containing particles obtained in Preparation Example 1 are shown in Fig. 1. In the figure, the scale bar shows 200 nm.

From the results shown in Fig. 1, it can be seen that the diameter of the gadolinium oxide-containing particles is about 20 nm.

### Preparation Example 2

A mixed aqueous solution was prepared by dissolving 2 mg of sodium lauryl sulfate and 4 mg of sodium persulfate in 1 mL of water.

### Example 1

To 1700 µL of the dispersion of the gadolinium oxide-containing particles obtained in Preparation Example 1, in which the concentration of the gadolinium was 16 mM, 100 µL of heptane was added, followed by heating at 75°C for 1 hour to condense, to give a condensate. To the condensate, 90 mg of methyl methacrylate and 10 mg of trimethylolpropane trimethacrylate were added, to give a monomer component-containing mixture which contained a monomer component and the gadolinium oxide-containing particles. To the monomer component-containing mixture obtained in the above, 1000 g of the mixed aqueous solution obtained in Preparation Example 2 was added, to give a mixed aqueous solution. A series of operations including irradiating the mixed aqueous solution with ultrasonic waves for one minute at 150W under ice-cooling by using an ultrasonic homogenizer manufactured by NISSEI Corporation under the trade name of US 300T, and then terminating the irradiation of ultrasonic waves for one minute was repeated 5 times, to give an emulsion. The emulsion was heated at 70°C for 4 hours, to polymerize the monomer components included in the emulsion. A dialysis was conducted to the resulting reaction solution for two days with a dialysis membrane [manufactured by SPECTRUM LABORATORIES, INC., trade name: Float-A-Lyzer G2, MWCO: 1000 kDa], to remove impurities remaining in the reaction solution, and thereby a composite particle dispersion A was obtained.

### (Determination of volume average particle diameter)

The composite particle dispersion A was suspended in ultrapure water, to give a measurement sample. The particle size distribution of the composite particles contained in the measurement sample was determined at 25°C by using a dynamic light scattering particle size distribution measuring device manufactured by NIKKISO CO., Ltd. under the trade name of NANO-TRACK UPA-EX 150.

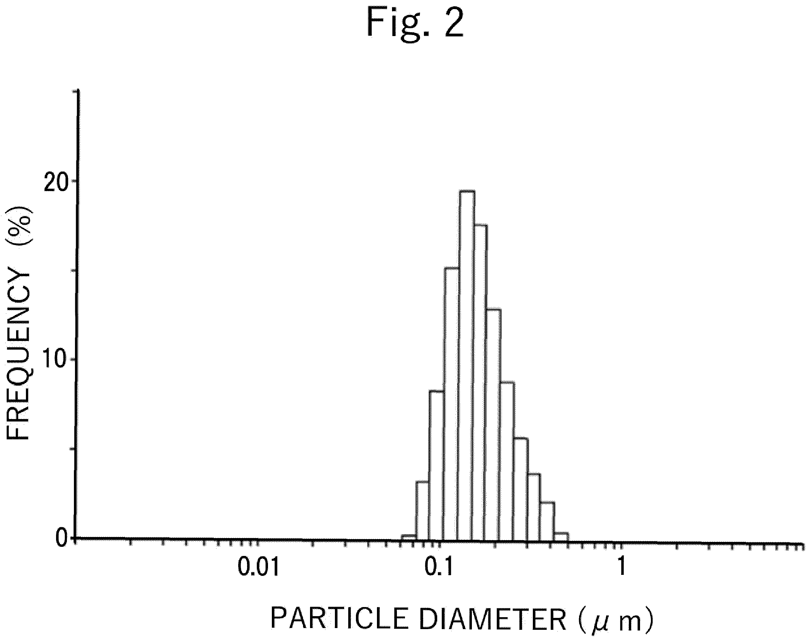

The particle size distribution of the composite particles obtained in Example 1 is shown in Fig. 2. From the results shown in Fig. 2, it can be seen that the volume average particle diameter of the composite particles is 165 nm.

### (Determination of content of gadolinium oxide)

The content of gadolinium oxide in the composite particles obtained in Example 1 was measured at 500°C by using a simultaneous thermogravimetric analyzer produced by HITACHI HIGH-TECH CORPORATION under the trade name of STA 7300. As a result, the content of gadolinium oxide in the composite particles obtained in Example 1 was 9. 6% by mass.

### (Observation of composite particles)

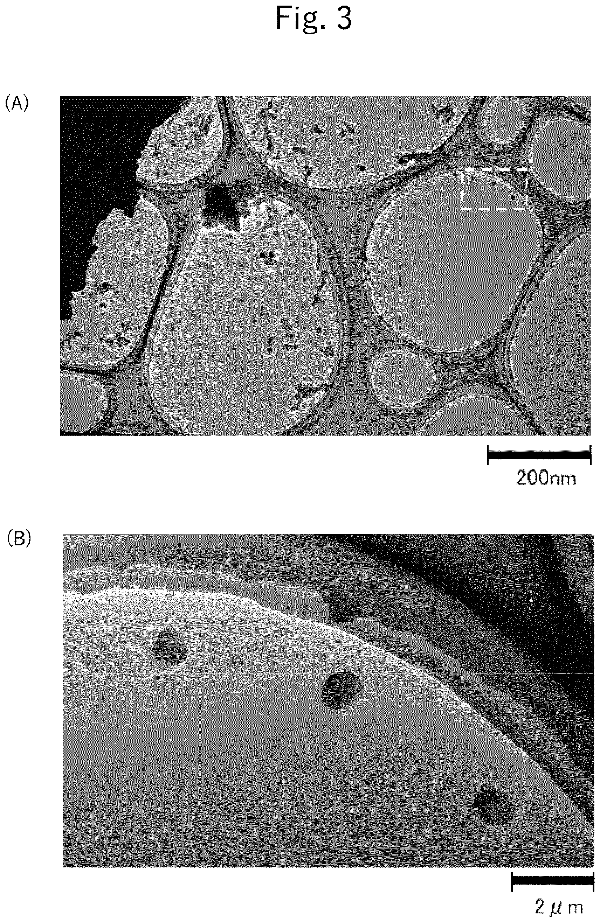

The composite particle dispersion A was diluted 1000 times with a solvent for dilution [components: 1 M nitric acid and 1% by mass of polyoxyethylene (10) octylphenyl ether (Triton X -100)], to give a measurement sample. The composite particles included in the measurement sample were observed at an acceleration voltage of 100 kV using a transmission electron microscope [made by JEOL Ltd., trade name: JEM-1400]. The result of observation of the composite particles obtained in Example 1 is shown in Fig. 3(A), and an enlarged area surrounded by a dashed line of Fig. 3(A) is shown in Fig. 3(B). In Fig. 3(A), a scale bar shows 200 nm. In Fig. 3(B), a scale bar shows 2 µm.

From the results shown in Fig. 3, it can be seen that the composite particles obtained in Example 1 have a coating film containing a polymer of methyl methacrylate and trimethylolpropane trimethacrylate on the surface of the gadolinium oxide particles.

### EXAMPLE 2

In Example 1, the same procedure as in Example 1 was carried out except that 90 mg of styrene and 10 mg of divinylbenzene were used instead of 90 mg of methyl methacrylate and 10 mg of trimethylolpropane trimethacrylate, to a composite particle dispersion B.

### (Determination of volume average particle diameter)

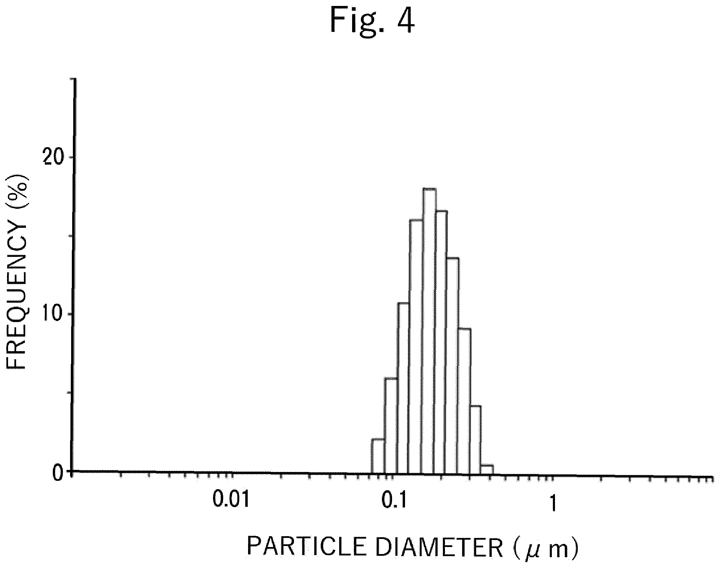

In Example 1, the same procedure as in Example 1 was carried out except that the composite particle dispersion B was used instead of the composite particle dispersion A, and the volume average particle diameter of the composite particles obtained in Example 2 was determined. The particle size distribution of the composite particles obtained in Example 2 is shown in Fig. 4. From the results shown in Fig. 4, it can be seen that the volume average particle size of the composite particles obtained in Example 2 is 175 nm.

### (Determination of content of gadolinium oxide)

In Example 1, the same operation as in Example 1 was carried out except that the composite particle dispersion B was used instead of the composite particle dispersion A, and the content of gadolinium oxide in the composite particles obtained in Example 2 was determined. As a result, the gadolinium oxide content in the composite particles B was 8. 3% by mass.

### Test example 1

The composite particle dispersion A obtained in Example 1 was added to a saline so that the concentration of the composite particles obtained in Example 1 was 0.0625 mM, 0.125 mM, 0.25 mM, 0.5 mM or 1 mM, to give a test sample (Experiment No. 1). The same procedures as in the above were carried out except that 10 % by volume of FBS-containing saline was used instead of the saline, to give a serum-containing test sample (Experiment No. 2).

A T-1 emphasized image of the test sample or the serum-containing test sample was photographed by using an MRI apparatus for small animals (manufactured by BRUKER BIOSPIN, INC., trade name: Biospec 70/20 USR). The T-1 emphasized image was photographed at 7T of external magnetic field intensity and at room temperature under the conditions of TR: 200 ms, TE: 6. 22 ms, FOV: 40 × 40 mm², matrix size: 256 × 256, NA: 2, and sliced thickness: 2 mm in accordance with a spin echo method.

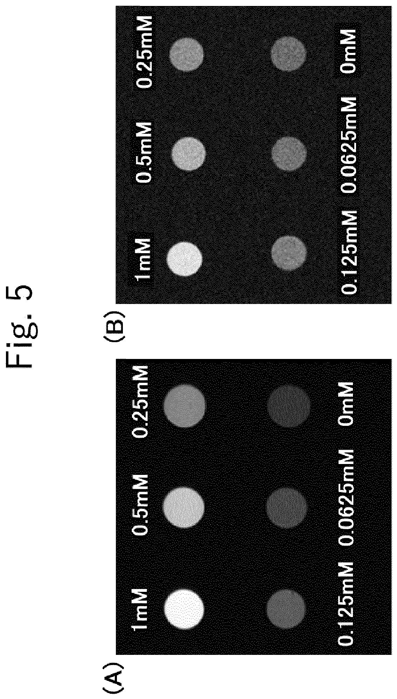

The T1 enhancement image in the presence of the test sample obtained in Test Example 1 is shown in Fig. 5(A), and the T1 enhancement image in the presence of the serum-containing test sample obtained in Test Example 1 is shown in Fig. 5(B).

From the results shown in Fig. 5A, it can be seen that the MR signal strength in the T1-enhanced image in the presence of the composite particles obtained in Example 1 (concentration of composite particle: 0.0625 mM to 1 mM) is significantly higher in comparison with the MR signal strength in the T1-enhanced image in the absence of the composite particles (concentration of composite particle: 0 mM) obtained in Example 1. In addition, from the results shown in Fig. 5B, it can be seen that the MR signal strength in the T1-enhanced image in the presence of the composite particles obtained in Example 1 (concentration of composite particle: 0.0625 mM to 1 mM) is significantly higher in comparison with the MR signal strength in the T1-enhanced image in the absence of the composite particles (concentration of composite particle: 0 mM) obtained in Example 1, even though a serum is included in the test sample, as in living bodies. Thus, it can be seen that the composite particles obtained in Example 1 can be used as a positive MRI contrast agent.

Incidentally, when the composite particle dispersion B obtained in Example 2 was used instead of the composite particle dispersion A obtained in Example 1, the same tendency as in the case of the composite particle dispersion A obtained in Example 1 is observed. Thus, it can be seen that the composite particles obtained in Example 2 can be used as a positive MRI contrast agent.

From the above results, it can be seen that the composite particles according to the present invention can be used as a positive MRI contrast agent.

### Comparative example 1

A 250 mL bottle made of polypropylene was charged with 500 mg of hexadecyltrimethylammonium bromide, 4000 mg of a nonionic surfactant [produced by SIGMA-ALDRICH, trade name: Pluronic F -127], 33.7 mL of ethanol, 10.66 mL of aqueous ammonia having a concentration of 28% by mass and 95.96 mL of ultrapure water. The bottle made of polypropylene was capped, and the bottle was stirred at 25°C for 8 hours at a rotation speed of 900 to 1000 min⁻¹, to give a micelle solution.

To the micelle solution, 1690 mg of tetraethylorthosilicate was added. The resulting mixture was stirred at 25°C for 1 minute at a high speed. The mixture after stirring was allowed to stand at 25°C for 24 hours, to give 8 mL of a white solution. The resulting white solution was centrifuged at 40000 ×g for 20 minutes, to precipitate particles.

The precipitated particles were dispersed in 8 mL of ultrapure water, to give a particle dispersion. The particle dispersion was stirred for 10 minutes, and then the particle dispersion was irradiated with ultrasonic waves for 20 minutes. The particle dispersion after the irradiation of ultrasonic waves was conducted to centrifugation at 40000 ×g for 20 minutes, to precipitate the particles. Thereafter, a series of operations including dispersion of the particles in ultrapure water, stirring of the resulting particle dispersion, irradiation of ultrasonic waves to the particle dispersion and sedimentation of the particles was carried out twice in the same manner as described above.

Thereafter, the particles were washed once with ethanol in the same manner when the ultrapure water was used, except that ethanol was used instead of the ultrapure water. The washed particles were placed in an oven maintained to 65°C, and dried for 12 hours. The dried product obtained in the above was pulverized with an agate mortar, to give a powder. The powder obtained in the above was placed in an electric furnace, and calcined at 550°C for 5 hours, to give 180 mg of silica nanoparticles.

### (Determination of volume average particle diameter)

The silica nanoparticles were suspended in ultrapure water, to give a measurement sample. The particle size distribution of the particles included in the measurement sample was determined at 25°C by using a dynamic light scattering particle size distribution measuring apparatus [manufactured by MALVERN PANALYTICAL LTD., product name: ZETASIZER NANO ZS]. As a result, the volume average particle size of the silica nanoparticles was 0.143 µm.

### Test example 2

A cell growth medium was prepared by adding FBS, penicillin and streptomycin to a Dulbecco's Modified Eagle Medium so that a concentration of the FBS was 10% by volume, a concentration of the penicillin was 100 units/mL, and a concentration of the streptomycin was 100 µg/mL.

In the cell growth medium, macrophage cell line RAW 264 was dispersed so that a concentration of the macrophage cell line was 1. 0 × 10⁵ cells/mL, to give a cell dispersion. The cell dispersion in an amount of 3 mL was seeded on wells of a 6-well plate. The cells after seeding were adhered to the wells of the 6-well plate by culturing the cells at 37°C for 24 hours in a 5% by volume carbon dioxide atmosphere.

The composite particles obtained in Example 1 was placed in the wells so that the concentration of the composite particles was 0.1 mg/mL. The composite particles in the wells were incubated at 37°C for 24 hours in a 5% by volume carbon dioxide atmosphere. The cells were washed three times with 1 mL of PBS. After washing the cells, 500 µL of a solution containing 0.25% by volume of trypsin and 1mM of EDTA was added to the cells, and thereafter the resulting mixture was allowed to stand for 5 minutes at 37°C in a 5 % by volume carbon dioxide atmosphere to suspend the cells, thereby a cell-suspending solution was obtained. To the cell-suspending solution, 500 µL of the cell growth medium was added, to give a cell suspension. The cell suspension was added to a 15 mL centrifuge tube (Spitz tube). The centrifuge tube including the cell suspension was centrifuged at 1000 rpm (180 ×g) for 5 minutes, and a supernatant was removed from the cell suspension, to give a cell mass containing the composite particles.

To the cell mass obtained in the above, 150 µL of cell lysis solution [components: 2 % by mass Triton X-100 and 2N nitric acid] was added, and thereafter the cells were dissolved by pipetting, to give a sample. The amount of the gadolinium contained in the sample was determined by using a polarized Zeeman atomic absorption spectrophotometer, and thereby the amount of the composite particles taken up per cell was determined. As a result, the amount of the composite particles taken up per cell was found to be 632 to 1201 pg.

In addition, the same procedures as in the case when the composite particles obtained in Example 1 was used were carried out, except that the composite particles obtained in Example 1 were used instead of the composite particles obtained in Comparative Example 1, and the amount of silica nanoparticles taken up per cell was determined. As a result, the amount of the composite particles taken up per cell was found to be 4.72 to 8.63 pg.

From these results, it can be seen that the amount of the composite particles uptaken into the macrophage cell line RAW 264 in Example 1 is greater than the amount of the silica nanoparticles uptaken into the macrophage cell line RAW 264 in Comparative Example 1. Accordingly, it can be seen that the composite particles obtained in Example 1 are easily incorporated into the macrophages in comparison with the silica nanoparticles.

Incidentally, the same procedures as the above when the composite particles obtained in Example 1 was used were carried out except that the composite particle dispersion B obtained in Example 2 was used instead of the composite particle dispersion A obtained in Example 1. In this case, it can be seen that the composite particles B obtained in Example 2 tend to be easily incorporated into the macrophages as in the case of the composite particles obtained in Example 1.

In addition, the same procedures when the composite particles obtained in Example 1 was used in the above were carried out, except that the composite particles made of gadolinium oxide particles and gelatin or dextran in which each surface of the gadolinium oxide particles was covered with the gelatin or the dextran were used instead of the composite particle dispersion A obtained in Example 1. In this case, it can be seen that the composite particles tend to be hardly incorporated into the macrophages as in the case of the silica nanoparticles obtained in Comparative Example 1.

From these results, it can be seen that the composite particles according to the present invention are efficiently incorporated into the macrophages.

### Preparation Example 3

A special feed was prepared by nixing sodium chloride and a collagen cross-linked enzyme inhibitor (3-aminopropionitrile) with a rat feed commercially available from Oriental Yeast Co., ltd., under the tradename of Experimental animal feed MF so that the concentration of sodium chloride was 8% by mass, and the concentration of the collagen cross-linked enzyme inhibitor was 0.12% by mass.

### Preparation Example 4

A left total carotid artery of a seven weeks-old male Sprague Dawley rat was cut under systemic anesthesia. The left total carotid artery was anastomosed to the right total carotid artery by end anastomosis to give a new blood vessel branch. The rat after postoperative was bred for one month with the special feed obtained in Preparation Example 3.

After breeding, the blood vessel branch of the rat was observed. As a result, it has been found that an aneurysm was formed on the blood vessel branch. In addition, the blood vessel branch was imaged by means of a 7T-MRI Scanner [manufactured by Bruker BioSpin GmbH, trade name: 7T MRI/MRS System BioSpec 70/ 20 USR for animals], and the image was observed. As a result, it has been found that a sac-shaped aneurysm was formed on the blood vessel branch.

A tissue specimen was prepared by ablating an aneurysm lesion. An immunohistochemical staining of the tissue specimen of the aneurysm lesion was performed by using anti-CD68 antibody and anti-α-SMA antibody. Incidentally, CD 68 is a marker of macrophages, and α-SMA is a marker of smooth muscle cells.

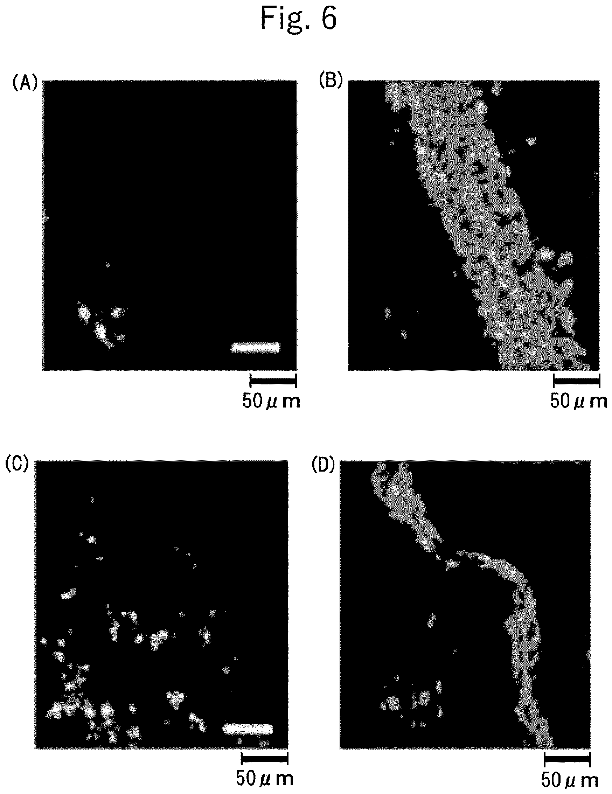

Fig. 6(A) shows the result of immunohistochemical staining of normal carotid artery using the anti-CD68 antibodies in Preparation Example 4. Fig. 6(B) shows the result of immunohistochemical staining of normal carotid artery using the anti-α-SMA antibodies in Preparation Example 4. Fig. 6(C) shows the result of immunohistochemical staining of aneurysm lesions using the anti-CD68 antibodies in Preparation Example 4. Fig. 6(D) shows the result of immunohistochemical staining of aneurysm lesions using the anti-α-SMA antibodies in Preparation Example 4. In each figure, the scale bar shows 50 µm.

From the results shown in Fig. 6, it can be seen that the number of macrophages which are CD68 positive cells contained in the aneurysm lesion [Fig. 6(C)] is greater than the number of macrophages contained in the normal carotid artery [Fig. 6(A)]. From these results, it can be seen that macrophages are infiltrated in aneurysm lesions. In addition, it can be seen that the number of smooth muscle cells which are α-SMA positive cells contained in aneurysm lesions [Fig. 6(D)] is less than the number of smooth muscle cells contained in normal carotid artery [Fig. 6(B)]. From these results, it can be seen that the smooth muscle cell layer of the blood vessel is thinned in aneurysm lesions. The infiltration of macrophages and thinning of smooth muscle cell layers are characteristic findings of cerebral aneurysm lesions. Accordingly, it can be seen that the rats obtained in Preparation Example 4 can be used as an aneurysm model animal.

### Example 3

A macrophage imaging agent was prepared by adding the composite particle dispersion A obtained in Example 1 to saline so that the concentration of the composite particles was 10 mg/mL.

### Test Example 3

The macrophage imaging agent obtained in Example 3 in an amount of 200 µL was administered to a tail vein of a 6 weeks old female BALB/c mouse. The T1 emphasized image of the whole body of the mouse was imaged by means of an MRI Scanner for small animals [manufactured by Bruker BioSpin GmbH, trade name: BioSpec 70/ 20 USR] with the passage of time, and the contrast of the T1 enhanced image was observed over time. The T1 emphasized image was photographed in accordance with a spin echo method at an external magnetic field intensity of 7T and at room temperature under the conditions of TR: 200 ms, TE: 6. 22 ms, FOV: 80 × 40 mm², matrix size: 256 × 128, NA: 2, slice thickness: 2 mm, number of sliced sheets: 9 sheets, and fat suppression.

Fig. 7(A) shows a T1-enhanced image of a mouse prior to administration of a macrophage imaging agent obtained in Example 3. Fig. 7(B) shows a T1-enhanced image of a mouse after 30 minutes passed from the end of administration of the macrophage imaging agent obtained in Example 3. Fig. 7(C) shows a T1-enhanced image of a mouse after one hour passed from the end of administration of the macrophage imaging agent obtained in Example 3. Fig. 7(D) shows a T1-enhanced image of a mouse after 2 hours passed from the end of administration of the macrophage imaging agent obtained in Example 3. Fig. 7(E) shows a T1-enhanced image of a mouse after 24 hours passed from the end of administration of the macrophage imaging agent obtained in Example 3. In the figures, an arrow indicates a liver of a mouse.

From the results shown in Fig. 7, it can be seen that the T1 contrast of the liver is increased in each of the T1-enhanced images after one hour, 2 hours and 24 hours passed from the end of administration of the macrophage imaging agent obtained in Example 3, in comparison with the T1-enhanced image of the liver before administration of the macrophage imaging agent obtained in Example 3. Accordingly, it can be seen that the macrophage imaging agent obtained in Example 3 can be used as a positive MRI contrast agent because the macrophage imaging agent emits a positive MR signal in living bodies.

### Example 4

A macrophage imaging agent was prepared by adding the composite particle dispersion A obtained in Example 1 to saline so that the concentration of the composite particles was 40 mg/mL.

### Test Example 4

The macrophage imaging agent obtained in Example 4 in an amount of 1mL was administered to a tail vein of a rat (aneurysm model animal) obtained in Preparation Example 4. The T1 emphasized image of a neck and a dome of a carotid artery of the rat was imaged by means of an MRI Scanner for small animals [manufactured by Bruker BioSpin GmbH, trade name: BioSpec 70/ 20 USR] with the passage of time, and the T1 contrast of the T1 enhanced image was observed over time. The T1 emphasized image was photographed in accordance with a gradient echo method accompanying a black-blood method at an external magnetic field intensity of 7T and at room temperature under the conditions of TR: 750 ms, TE: 3.2 ms, flip angle: 90°, FOV: 32 × 32 mm², matrix size: 128 × 128, NA: 4, slice thickness: 0.5 mm, number of sliced sheets: 5 sheets, black-blood inversion time: 700 ms, and black-blood slab thickness: 5.5 mm.

Fig. 8(A) shows T1-enhanced images on a neck side of a carotid artery of a rat prior to administration of a macrophage imaging agent obtained in Example 4. Fig. 8(B) shows T1-enhanced images on a neck side of a carotid artery of a rat after 24 hours passed from the end of administration of the macrophage imaging agent obtained in Example 4. Fig. 8(C) shows T1-enhanced images on a dome side of a carotid artery of a rat prior to administration of a macrophage imaging agent obtained in Example 4. Fig. 8(D) shows T1-enhanced images on a dome side of a carotid artery of a rat after 24 hours passed from the end of administration of the macrophage imaging agent obtained in Example 4. In each figure, a scale bar shows 1 mm.

From the results shown in Fig. 8, it can be that the T1 contrast in the periphery of the carotid aneurysm is increased in the T1 enhancement images after 24 hours passed from administration of the macrophage imaging agent obtained in Example 4 [Fig. 8(B) and Fig. 8(D)] in comparison with the T1-enhanced image prior to administration of the macrophage imaging agent obtained in Example 4 [Fig. 8(A) and Fig. 8(C)]. From these results, it can be seen that the macrophage imaging agent obtained in Example 4 makes it possible to visualize an aneurysm because the macrophage imaging agent is incorporated into the macrophages integrated to an aneurysm.

### Test Example 5

A cell growth medium was prepared by adding FBS, penicillin and streptomycin to a Dulbecco's Modified Eagle Medium so that a concentration of the FBS was 10% by volume, a concentration of the penicillin was 100 units/mL, and a concentration of the streptomycin was 100 µg/mL.

A cell dispersion was prepared by dispersing cells of mouse-derived fibroblast strain L929 in the cell growth medium so that the concentration of the cells was 3. 2 × 10⁴ /mL. The cell dispersion in an amount of 100 µL was seeded in wells of a 96 well plate. The cells after seeding were cultured at 37°C for 24 hours in a 5 % by volume carbon dioxide atmosphere, to adhere the cells to the wells of the 96 well plate.

The composite particle dispersion A obtained in Example 1 was dispersed in 10 µL of ultrapure water so that the concentration of the composite particles was 0.00001 to 0.1 mg/mL, to give a composite particle dispersion. The composite particle dispersion obtained in the above in an amount of 10 µL was added to a sample containing the cells in the wells, and thereafter the resulting sample containing the composite particles and the cells in the wells was incubated at 37°C for 48 hours in a 5 % by volume carbon dioxide atmosphere. The incubated sample was washed with 0.1 mL of PBS three times. To the sample after washing, 10 µL of a CCK-8 solution [manufactured by Doujin Laboratories, product name: Cell Counting Kit-8] and 100 µL of the cell growth medium were added, and thereafter the resulting mixture was incubated at 37°C for 1. 5 hours in a 5 % by volume carbon dioxide atmosphere, to perform a color reaction. The 96 well plate including the incubated sample was put in a microplate reader [manufactured by Bio-Rad Laboratories, Inc., product name: iMark], and an absorbance of the incubated sample in each well at 450 nm (hereinafter referred to as "absorbance A") was determined (reference wavelength: 600 nm).

In addition, the same procedures as the above when 10 µL of the composite particle dispersion was used were caried out except that 10 µL of ultrapure water was used instead of 10 µL of the composite particle dispersion, and an absorbance of the incubated sample in each well at 450 nm (hereinafter referred to as "absorbance B") was determined (reference wavelength: 600 nm).

A cell viability was calculated by dividing the absorbance A by the absorbance B.

In addition, the same procedures as the above when 10 µL of the composite particle dispersion was used were caried out except that10 µL of a contrast agent for clinical use (clinical contrast agent) [manufactured by Bayer Yakuhin, Ltd., trade name: Magnevist (registered trademark)] was used instead of 10 µL of the composite particle dispersion, and an absorbance of the incubated sample in each well at 450 nm (hereinafter referred to as "absorbance C") was determined (reference wavelength: 600 nm).

A cell viability was calculated by dividing the absorbance C by the absorbance B.

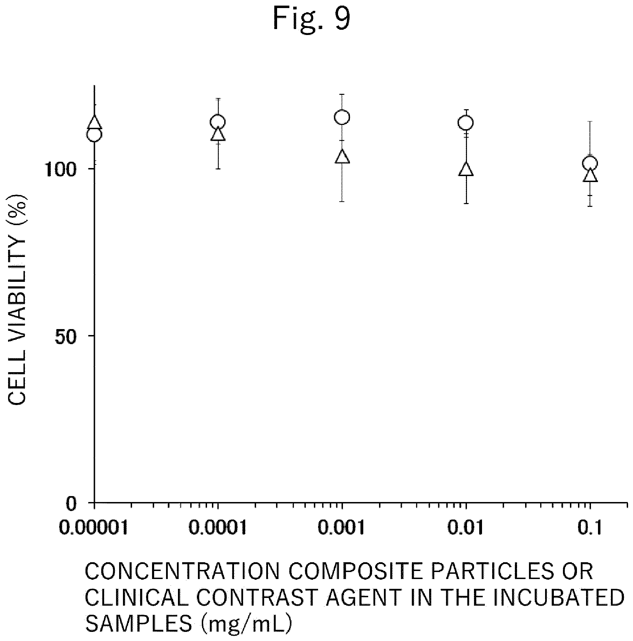

The relation between the concentration of the composite particles in the incubated sample obtained in the above or the concentration of the contrast agent for clinical use in the incubated sample obtained in the above and the cell viability is shown in Fig. 9. In Fig. 9, a white circle shows a relation between the concentration of the composite particles in the incubated sample and the cell viability, and a white triangle shows a relation between the concentration of the clinical contrast agent in the incubated sample and the cell viability.

From the results shown in Fig. 9, it can be seen that the cell viability when the composite particles obtained in Example 1 were used is the same level as the cell viability when the contrast agent for clinical use [manufactured by Bayer Yakuhin, Ltd., trade name: Magnevist (registered trademark)] was used. From this result, it can be seen that the composite particles obtained in Example 1 shows a low load on living bodies.

Incidentally, when the same procedures as employed the composite particles obtained in Example 1 were carried out except that the composite particles obtained in Example 2 was used instead of the composite particles obtained in Example 1, it can be seen that the composite particles obtained in Example 2 shows a tendency that a load on the living body tends is low as in the case of the composite particles obtained in Example 1.

As described above, according to the composite particles of the present invention, a macrophage can be visualized. In addition, according to the composite particles of the present invention, an aneurysm can be visualized by visualizing the macrophage. Furthermore, according to the composite particles of the present invention, a load on living bodies can be reduced. Accordingly, it is expected that the composite particles according to the present invention and the macrophage imaging agent containing the composite particles according to the present invention are suitably used in applications such as image diagnosis of diseases involving a macrophage, determination of an effect of drug therapy on diseases, and the like.

## Claims

1. A macrophage imaging agent used for macrophage imaging by a magnetic resonance imaging method, comprising composite particles in which a coating film containing a polymer made by polymerizing a monomer component containing a monomer having an ethylenically unsaturated bond is formed on the surface of a gadolinium oxide-containing particle.

2. A composite particle used in a macrophage imaging agent, comprising a gadolinium oxide-containing particle and a coating film containing a polymer made by polymerizing a monomer component containing a monomer having an ethylenically unsaturated bond, wherein the coating film is formed on the surface of the gadolinium oxide-containing particle.

3. A method for producing the composite particle according to claim 2, comprising:
mixing a monomer component comprising a monomer having an ethylenically unsaturated bond with a gadolinium oxide-containing particle,
emulsifying the resulting monomer component-containing mixture in water in the presence of a surfactant and a polymerization initiator, and
thereafter polymerizing the monomer component to form a coating film on the surface of the gadolinium oxide-containing particle.
